(19) Europäisches Patentamt
European Patent Office
Office européen des brevets

(11) Veröffentlichungsnummer: **0 016 974**
**A1**

(12) # EUROPÄISCHE PATENTANMELDUNG

(21) Anmeldenummer: **80101041.4**

(22) Anmeldetag: **03.03.80**

(51) Int. Cl.³: **C 07 D 213/30**, C 07 D 213/50, A 01 N 43/40

(30) Priorität: **09.03.79 DE 2909287**

(43) Veröffentlichungstag der Anmeldung: **15.10.80**
**Patentblatt 80/21**

(84) Benannte Vertragsstaaten: **AT BE CH DE FR GB IT NL SE**

(71) Anmelder: **BAYER AG, Zentralbereich Patente, Marken und Lizenzen, D-5090 Leverkusen 1, Bayerwerk (DE)**

(72) Erfinder: **Holmwood, Graham, Dr.,**
**Katernbergerstrasse 184, D-5600 Wuppertal 1 (DE)**
Erfinder: **Frohberger, Paul-Ernst, Dr.,**
**Willi-Baumeister-Strasse 5, D-5090 Leverkusen (DE)**

(54) **3-Substituierte Pyridin-Derivate, Verfahren zu ihrer Herstellung, ihre Verwendung als Fungizide und Zwischenprodukte.**

(57) Verbindungen der Formel

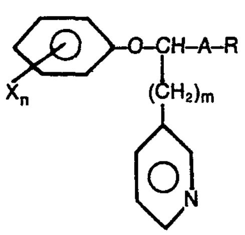

(I)

in welcher
A für die –CO– oder die –CH(OH)–Gruppe steht,
R für Alkyl oder gegebenenfalls substituiertes Phenyl steht,
X für Halogen, Alkyl, Cycloalkyl, Alkoxy, Alkylthio, Halogenalkyl, gegebenenfalls substituiertes Phenyl, gegebenenfalls substituiertes Phenoxy, gegebenenfalls substituiertes Phenylalkyl oder gegebenenfalls substituiertes Phenylalkoxy steht,
n für ganze Zahlen von 0 bis 3 steht und
m für 0 oder 1 steht,
sowie deren physiologisch verträgliche Säureadditions-Salze und Metallsalz-Komplexe.

Verfahren zu ihrer Herstellung durch Umsetzung von Halogen-pyridin-ketone der Formel

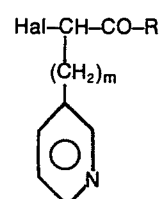

mit Phenolen der Formel

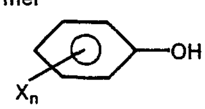

oder durch Sättigung von $\alpha,\beta$-ungesättigte Ketone der Formel

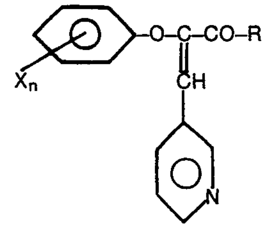

(IV)

und gegebenenfalls durch Reduktion der Keto-Derivate zu den sekundären Alkoholen.

EP 0 016 974 A1

0016974

BAYER AKTIENGESELLSCHAFT
Zentralbereich
Patente, Marken und Lizenzen

5090 Leverkusen, Bayerwerk
Slr/Kü
Ib

**BEZEICHNUNG GEÄNDERT
siehe Titelseite**

3-Substituierte Pyridin-Derivate, Verfahren zu ihrer Herstellung sowie ihre Verwendung als Fungizide

Die vorliegende Erfindung betrifft neue 3-substituierte Pyridin-Derivate, mehrere Verfahren zu ihrer Herstellung sowie ihre Verwendung als Fungizide.

Es ist bereits bekannt geworden, daß Zink-ethylen-1,2-bisdithiocarbamidat ein gutes Mittel zur Bekämpfung von pilzlichen Pflanzenkrankheiten ist (vergleiche Phytopathology 33, 1113 (1963)). Jedoch ist dessen Einsatz nur beschränkt möglich, da es bei niedrigen Aufwandmengen und -konzentrationen nicht immer eine ganz befriedigende Wirkung zeigt.

Es wurden neue 3-substituierte Pyridin-Derivate der Formel

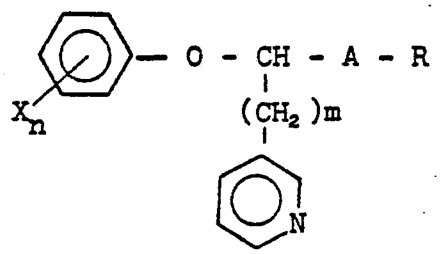

(I)

in welcher

A für die -CO- oder die -CH(OH)-Gruppe steht,

R für Alkyl oder gegebenenfalls substituiertes Phenyl steht,

X für Halogen, Alkyl, Cycloalkyl, Alkoxy, Alkylthio, Halogenalkyl, gegebenenfalls substituiertes Phenyl, gegebenenfalls substituiertes Phenoxy, gegebenenfalls substituiertes Phenylalkyl oder gegebenenfalls substituiertes Phenylalkoxy steht,

n für ganze Zahlen von 0 bis 3 steht und

m für 0 oder 1 steht,

sowie deren physiologisch verträglichen Säureadditions-Salze und Metallsalz-Komplexe gefunden.

Die Verbindungen der Formel (I) besitzen ein asymmetrisches Kohlenstoffatom; sie können deshalb in den beiden optischen Isomeren oder als Racemate vorliegen. Sämtliche Isomeren werden erfindungsgemäß beansprucht.

Weiterhin wurde gefunden, daß man die 3-substituierten Pyridin-Derivate der Formel (I) erhält, wenn man

a) Halogen-pyridin-ketone der Formel

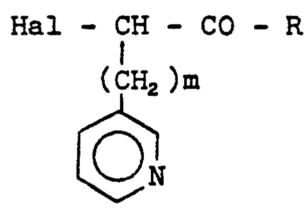

(II)

in welcher

R und m die oben angegebene Bedeutung haben und

Hal für Halogen, insbesondere Chlor oder Brom steht,

mit Phenolen der Formel

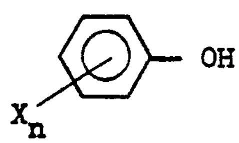

(III)

in welcher

X und n die oben angegebene Bedeutung haben,

in Gegenwart eines Verdünnungsmittels und in Gegenwart eines Säurebindemittels umsetzt, oder

b) α,ß-ungesättigte Ketone der Formel

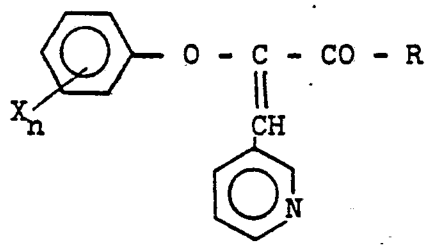

(IV)

in welcher

R,X und n die oben angegebene Bedeutung haben,

in an sich bekannter Art und Weise

α) mit Wasserstoff in Gegenwart eines Katalysators und gegebenenfalls in Gegenwart eines polaren Verdünnungsmittels oder

ß) mit einem Metall/Säure-Gemisch, wie insbesondere Zink/Eisessig umsetzt und

gegebenenfalls die nach den Verfahrensvarianten (a) und (b) erhaltenen Keto-Derivate der Formel I nach bekannten Methoden in üblicher Weise zu den sekundären Alkoholen der Formel I reduziert.

An die so erhaltenen Verbindungen der Formel (I) kann gegebenenfalls anschließend eine Säure oder ein Metallsalz addiert werden.

Die neuen 3-substituierten Pyridin-Derivate der Formel (I) weisen gute fungizide Eigenschaften auf.

Ueberraschenderweise zeigen die erfindungsgemäßen 3-substituierten Pyridin-Derivate der Formel (I) eine erheblich höhere fungizide Wirksamkeit, insbesondere gegen Mehltauarten, als das aus dem Stand der Technik bekannte Zink-ethylen-1,2-bisdithiocarbamidat, welches ein bekannter Stoff gleicher Wirkungsrichtung ist. Die erfindungsgemäßen Wirkstoffe stellen somit eine Bereicherung der Technik dar.

Die erfindungsgemäßen 3-substituierten Pyridin-Derivate sind durch die Formel (I) allgemein definiert. In dieser Formel steht R vorzugsweise für Alkyl mit 1 bis 4 Kohlenstoffatomen sowie für gegebenenfalls substituiertes Phenyl, wobei als Substituenten vorzugsweise infrage kommen: Halogen, Alkyl mit 1 bis 4 Kohlenstoffatomen und Alkoxy mit 1 bis 2 Kohlenstoffatomen. X steht vorzugsweise für Halogen; Alkyl, Alkoxy und Alkylthio mit jeweils 1 bis 4 Kohlenstoffatomen; Cycloalkyl mit 5 bis 7 Kohlenstoffatomen; Halogenalkyl mit bis zu 2 Kohlenstoff- und bis zu 5 gleichen oder verschiedenen Halogenatomen, wie insbesondere Fluor- und Chloratomen; sowie für gegebenenfalls durch Halogen oder Alkyl mit 1 bis 2 Kohlenstoffatomen substituiertes Phenyl, Phenoxy, Phenylalkyl mit 1 bis 2 Kohlenstoffatomen im Alkylteil oder Phenylalkoxy mit 1 bis 2 Kohlenstoffatomen im Alkylteil. Der Index n steht vorzugsweise für die ganzen Zahlen 0, 1 und 2. A und der Index m haben die in der Erfindungsdefinition angegebene Bedeutung.

Ganz besonders bevorzugt sind diejenigen Verbindungen der Formel (I), in denen R für tert.-Butyl, Isopropyl, Phenyl, Chlorphenyl, Fluorphenyl oder Dichlorphenyl steht; X für Fluor, Chlor, Brom, Methyl, Ethyl, Methoxy, Methylthio, Trifluormethyl, Cyclohexyl, Phenyl, Chlorphenyl, Phenoxy, Chlorphenoxy, Chlorbenzyl oder Chlorbenzyloxy steht; der Index n für die ganzen Zahlen 0, 1 und 2 steht; und A sowie der Index m die in der Erfindungsdefinition angegebene Bedeutung haben.

Im einzelnen seien außer den bei den Herstellungsbeispielen genannten Verbindungen die folgenden Verbindungen der allgemeinen Formel (I) genannt:

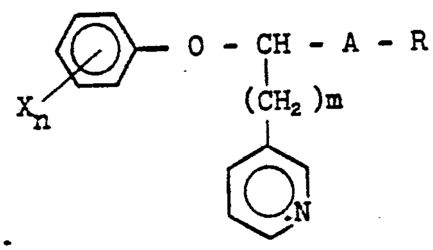

(I)

| $X_n$ | A | R | m |
|---|---|---|---|
| - | CO | $C(CH_3)_3$ | 0 |
| $4-OCH_3$ | CO | $C(CH_3)_3$ | 0 |
| 4-F | CO | $C(CH_3)_3$ | 0 |
| $4-CH_3$ | CO | $C(CH_3)_3$ | 0 |
| $3,4-Cl_2$ | CO | $C(CH_3)_3$ | 0 |
| $2-C_2H_5$ | CO | $C(CH_3)_3$ | 0 |
| 4-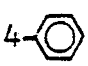 | CO | $C(CH_3)_3$ | 0 |
| 4-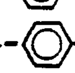-Cl | CO | $C(CH_3)_3$ | 0 |
| 4-O-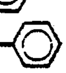 | CO | $C(CH_3)_3$ | 0 |
| 4-O-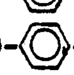-Cl | CO | $C(CH_3)_3$ | 0 |
| 4-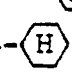 | CO | $C(CH_3)_3$ | 0 |
| $4-CH_2$-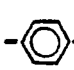-Cl | CO | $C(CH_3)_3$ | 0 |
| $4-O-CH_2$-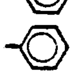-Cl | CO | $C(CH_3)_3$ | 0 |
| - | CH(OH) | $C(CH_3)_3$ | 0 |
| $2,4-Cl_2$ | CH(OH) | $C(CH_3)_3$ | 0 |
| $2-CH_3$ | CH(OH) | $C(CH_3)_3$ | 0 |
| $2-CH_3,4-Cl$ | CH(OH) | $C(CH_3)_3$ | 0 |
| 2-Cl | CH(OH) | $C(CH_3)_3$ | 0 |
| $4-O-CH_3$ | CH(OH) | $C(CH_3)_3$ | 0 |

| $X_n$ | A | R | m |
|---|---|---|---|
| 4-F | CH(OH) | $C(CH_3)_3$ | 0 |
| $4-CH_3$ | CH(OH) | $C(CH_3)_3$ | 0 |
| $3,4-Cl_2$ | CH(OH) | $C(CH_3)_3$ | 0 |
| $2-C_2H_5$ | CH(OH) | $C(CH_3)_3$ | 0 |
| 4-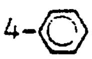 | CH(OH) | $C(CH_3)_3$ | 0 |
| 4-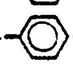-Cl | CH(OH) | $C(CH_3)_3$ | 0 |
| 4-O-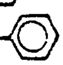 | CH(OH) | $C(CH_3)_3$ | 0 |
| 4-O-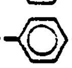-Cl | CH(OH) | $C(CH_3)_3$ | 0 |
| 4-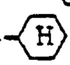 | CH(OH) | $C(CH_3)_3$ | 0 |
| $4-CH_2$-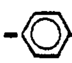-Cl | CH(OH) | $C(CH_3)_3$ | 0 |
| $4-O-CH_2$-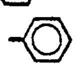-Cl | CH(OH) | $C(CH_3)_3$ | 0 |
| - | CO | $C(CH_3)_3$ | 1 |
| $4-OCH_3$ | CO | $C(CH_3)_3$ | 1 |
| 4-F | CO | $C(CH_3)_3$ | 1 |
| $2-C_2H_5$ | CO | $C(CH_3)_3$ | 1 |
| 4-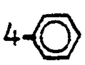 | CO | $C(CH_3)_3$ | 1 |
| 4-O-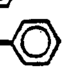 | CO | $C(CH_3)_3$ | 1 |
| 4-O-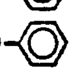-Cl | CO | $C(CH_3)_3$ | 1 |
| 4-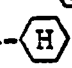 | CO | $C(CH_3)_3$ | 1 |
| $4-CH_2$-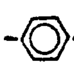-Cl | CO | $C(CH_3)_3$ | 1 |
| $4-O-CH_2$-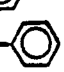-Cl | CO | $C(CH_3)_3$ | 1 |
| - | CH(OH) | $C(CH_3)_3$ | 1 |
| 4-Cl | CH(OH) | $C(CH_3)_3$ | 1 |
| $2,4-Cl_2$ | CH(OH) | $C(CH_3)_3$ | 1 |
| $2-CH_3$ | CH(OH) | $C(CH_3)_3$ | 1 |

| $X_n$ | A | R | m |
|---|---|---|---|
| 2-$CH_3$,4-Cl | CH(OH) | $C(CH_3)_3$ | 1 |
| 2-Cl | CH(OH) | $C(CH_3)_3$ | 1 |
| 4-$OCH_3$ | CH(OH) | $C(CH_3)_3$ | 1 |
| 4-F | CH(OH) | $C(CH_3)_3$ | 1 |
| 4-$CH_3$ | CH(OH) | $C(CH_3)_3$ | 1 |
| 3,4-$Cl_2$ | CH(OH) | $C(CH_3)_3$ | 1 |
| 2-$C_2H_5$ | CH(OH) | $C(CH_3)_3$ | 1 |
| 4-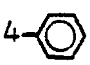 | CH(OH) | $C(CH_3)_3$ | 1 |
| 4-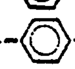-Cl | CH(OH) | $C(CH_3)_3$ | 1 |
| 4-O-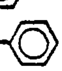 | CH(OH) | $C(CH_3)_3$ | 1 |
| 4-O-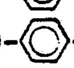-Cl | CH(OH) | $C(CH_3)_3$ | 1 |
| 4-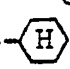 | CH(OH) | $C(CH_3)_3$ | 1 |
| 4-$CH_2$-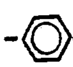-Cl | CH(OH) | $C(CH_3)_3$ | 1 |
| 4-O-$CH_2$-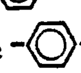-Cl | CH(OH) | $C(CH_3)_3$ | 1 |
| 4-Cl | CO | 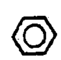 | 0 |
| 2,4-$Cl_2$ | CO | 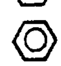 | 0 |
| 2-$CH_3$,4-Cl | CO | 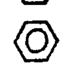 | 0 |
| 2-$CH_3$ | CO | 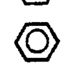 | 0 |
| 4-Cl | CH(OH) | 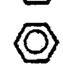 | 0 |
| 2,4-$Cl_2$ | CH(OH) | 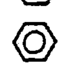 | 0 |
| 2-$CH_3$,4-Cl | CH(OH) | 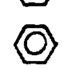 | 0 |
| 2-$CH_3$ | CH(OH) | 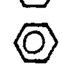 | 0 |
| 4-Cl | CO | 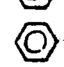 | 1 |
| 2,4-$Cl_2$ | CO | 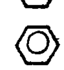 | 1 |
| 2-$CH_3$,4-Cl | CO | 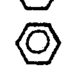 | 1 |
| 2-$CH_3$ | CO | 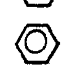 | 1 |

| $X_n$ | A | R | m |
|---|---|---|---|
| 4-Cl | CH(OH) | 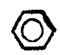 | 1 |
| 2,4-$Cl_2$ | CH(OH) | 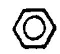 | 1 |
| 2-$CH_3$,4-Cl | CH(OH) | 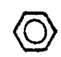 | 1 |
| 2-$CH_3$ | CH(OH) | 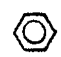 | 1 |
| 4-Cl | CO | 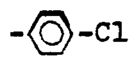 | 0 |
| 2,4-$Cl_2$ | CO | 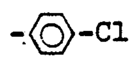 | 0 |
| 2-$CH_3$,4-Cl | CO | 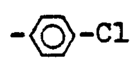 | 0 |
| 2-$CH_3$ | CO | 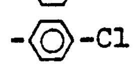 | 0 |
| 4-Cl | CH(OH) | 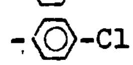 | 0 |
| 2,4-$Cl_2$ | CH(OH) | 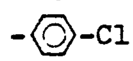 | 0 |
| 2-$CH_3$,4-Cl | CH(OH) | 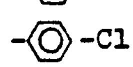 | 0 |
| 2-$CH_3$ | CH(OH) | 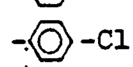 | 0 |
| 4-Cl | CO | 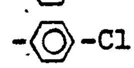 | 1 |
| 2,4-$Cl_2$ | CO | 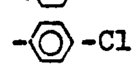 | 1 |
| 2-$CH_3$,4-Cl | CO | 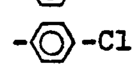 | 1 |
| 2-$CH_3$ | CO | 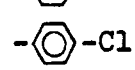 | 1 |
| 4-Cl | CH(OH) | 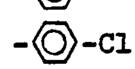 | 1 |
| 2,4-$Cl_2$ | CH(OH) | 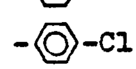 | 1 |
| 2-$CH_3$,4-Cl | CH(OH) | 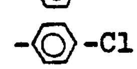 | 1 |
| 2-$CH_3$ | CH(OH) | 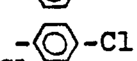 | 1 |
| 4-Cl | CO | 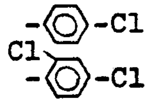 | 1 |
| 2,4-$Cl_2$ | CO | 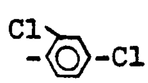 | 1 |
| 2-$CH_3$,4-Cl | CO | 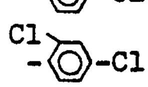 | 1 |
| 2-$CH_3$ | CO | 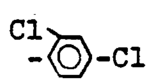 | 1 |

| $X_n$ | A | R | m |
|---|---|---|---|
| 4-Cl | CH(OH) | 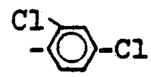 | 0 |
| 2,4-$Cl_2$ | CH(OH) | 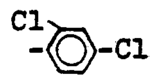 | 0 |
| 2-$CH_3$,4-Cl | CH(OH) | 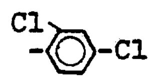 | 0 |
| 2-$CH_3$ | CH(OH) | 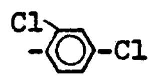 | 0 |
| 4-Cl | CO | 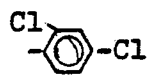 | 1 |
| 2,4-$Cl_2$ | CO | 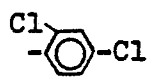 | 1 |
| 2-$CH_3$,4-Cl | CO | 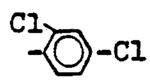 | 1 |
| 2-$CH_3$ | CO | 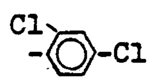 | 1 |
| 4-Cl | CH(OH) | 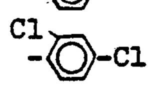 | 1 |
| 2,4-$Cl_2$ | CH(OH) | 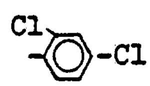 | 1 |
| 2-$CH_3$,4-Cl | CH(OH) | 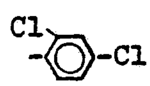 | 1 |
| 2-$CH_3$ | CH(OH) | 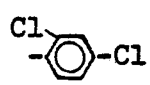 | 1 |

Verwendet man beispielsweise 1-Brom-3,3-dimethyl-1-pyridin-3-yl-butan-2-on und 4-Chlorphenol als Ausgangsstoffe, so kann der Reaktionsablauf durch das folgende Formelschema wiedergegeben werden (Verfahren a):

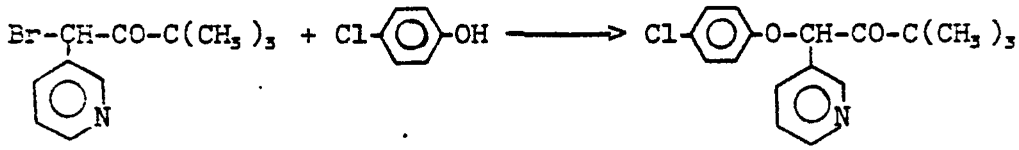

Verwendet man beispielsweise 2-(4-Chlorphenoxy)-4,4-dimethyl-1-pyridin-3-yl-1-penten-3-on und ein Gemisch von Zink/Essigsäure als Ausgangsstoffe, so kann der Reaktionsablauf durch das folgende Formelschema wiedergegeben werden (Verfahren b):

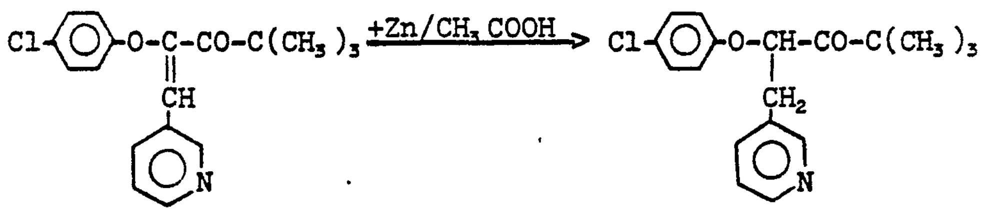


Verwendet man beispielsweise 1-(4-Chlorphenoxy)-3,3-dimethyl-1-pyridin-3-yl-butan-2-on und Natriumborhydrid als Ausgangsstoffe, so kann der Reaktionsablauf durch das folgende Formelschema wiedergegeben werden (Reduktion):

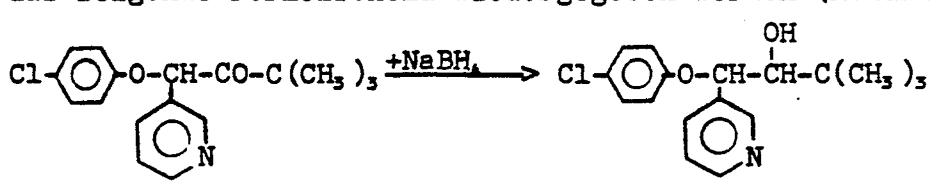


Die als Ausgangsstoffe für das Verfahren (a) zu verwendenden Halogen-pyridin-ketone sind durch die Formel (II) allgemein definiert. In dieser Formel stehen R und m vorzugsweise für diejenigen Reste, die bei der Beschreibung der erfindungsgemäßen Stoffe der Formel (I) bereits vorzugsweise für diese Substituenten genannt wurden.

Die Halogen-pyridin-ketone der Formel (II) sind noch nicht bekannt, können aber nach bekannten Verfahren hergestellt werden, indem man bei Pyridin-ketonen der Formel

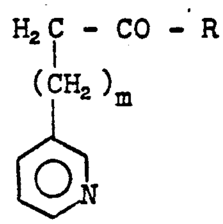

(V)

in welcher

R und m die oben angegebene Bedeutung haben,

eines der beiden aktiven Wasserstoffatome in üblicher Weise gegen Halogen austauscht (vergleiche auch die Herstellungsbeispiele).

Die Pyridin-ketone der Formel (V), in denen <u>m</u> für 0 steht, sind bekannt (vergleiche z.B. Synthesis <u>1975</u>, 705), bzw. lassen sich nach den dort beschriebenen Verfahren leicht herstellen, indem man z.B. entsprechende Carbonsäureester mit dem Lithiumderivat von 2-Picolin umsetzt. Die Pyridinketone der Formel (V), in denen <u>m</u> für 0 steht, können auch erhalten werden, wenn man 3-Pyridylessigester oder 3-Pyridylessigsäurenitril in allgemein bekannter Weise mit Grignard-Verbindungen vom Typ R-Mg-Hal umsetzt, wobei letztere Verbindungen in üblicher Weise aus den entsprechenden Halogeniden R-Hal und Magnesium erhalten werden.

Die Pyridin-ketone der Formel (V), in denen m für 1 steht, sind ebenfalls bekannt (vergleiche z.B. Arch.Pharm. 1974, 307, 550), bzw. lassen sich nach den dort beschriebenen Verfahren leicht herstellen, indem man das entsprechende Keton der Formel

$$H_3C - CO - R \qquad (VI)$$

in welcher

R die oben angegebene Bedeutung hat,

mit 3-Pyridinaldehyden umsetzt und das so erhaltene Produkt der Formel

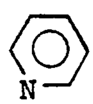 -CH = CH - CO - R (VII)

in welcher

R die oben angegebene Bedeutung hat,

mit Wasserstoff in Gegenwart eines Katalysators, wie vorzugsweise Raney-Nickel, reduziert (vergleiche auch die Herstellungsbeispiele).

Als Beispiele der Halogen-pyridin-ketone der Formel (II) seien genannt:

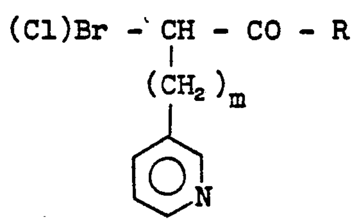

(IIa)

| R | m | R | m |
|---|---|---|---|
| $C(CH_3)_3$ | 0 | $C(CH_3)_3$ | 1 |
| $i-C_3H_7$ | 0 | $i-C_3H_7$ | 1 |
| 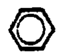 | 0 | 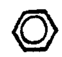 | 1 |
| 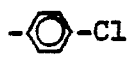 | 0 | 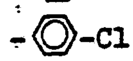 | 1 |
| 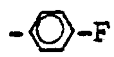 | 0 | 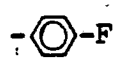 | 1 |
| 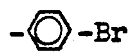 | 0 | 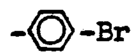 | 1 |
| 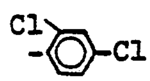 | 0 | 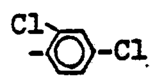 | 1 |
| 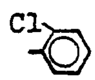 | 0 | 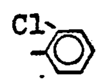 | 1 |
| 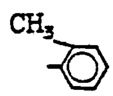 | 0 | 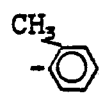 | 1 |
| 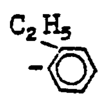 | 0 | 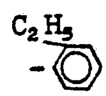 | 1 |
| 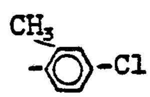 | 0 | 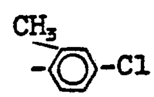 | 1 |
| 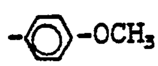 | 0 | 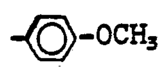 | 1 |
| 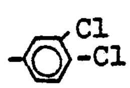 | 0 | 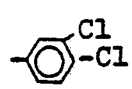 | 1 |
| 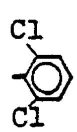 | 0 | 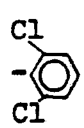 | 1 |
| 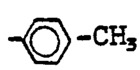 | 0 | 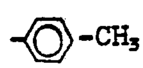 | 1 |

Die außerdem für das Verfahren (a) als Ausgangsstoffe zu verwendenden Phenole sind durch die Formel (III) allgemein definiert. In dieser Formel stehen X und n vorzugsweise für diejenigen Reste, die bei der Beschreibung der erfindungsgemäßen Stoffe der Formel (I) bereits vorzugsweise für diese Substituenten genannt wurden.

Die Phenole der Formel (III) sind allgemein bekannte Verbindungen der organischen Chemie. Als Beispiele seien genannt:
Phenol, 4-Chlorphenol, 4-Fluorphenol, 4-Methylphenol, 4-Methoxyphenol, 2-Chlorphenol, 2-Methylphenol, 2-Ethylphenol, 4-Phenylphenol, 4-Phenoxyphenol, 4-4'-Chlorphenylphenol, 4-4'-Chlorphenoxyphenol, 2,4-Dichlorphenol, 3,4-Dichlorphenol, 2,6-Dichlorphenol, 2-Methyl-4-chlorphenol.

Die als Ausgangsstoffe für das Verfahren (b) zu verwendenden α,β-ungesättigten Ketone sind durch die Formel (IV) allgemein definiert. In dieser Formel stehen R, X und n vorzugsweise für diejenigen Reste, die bei der Beschreibung der erfindungsgemäßen Stoffe der Formel (I) bereits vorzugsweise für diese Substituenten genannt wurden.

Die α,β-ungesättigten Ketone der Formel (IV) sind interessante neue Zwischenprodukte. Man erhält sie, wenn man Phenoxyketone der Formel

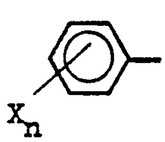 $O - CH_2 - CO - R$ (VIII)

in welcher

R, X und n die oben angegebene Bedeutung haben,

mit 3-Pyridinaldehyden der Formel

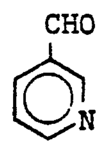

(IX)

in Gegenwart eines Verdünnungsmittels und in Gegenwart einer Base umsetzt.

Die Phenoxyketone der Formel VIII sind bekannte Verbindungen (vgl. hierzu DE-OS 2105 490 (Le A 13 458) bzw. US-PS 3812 142 und DE-OS 2201 063 (Le A 14 118) bzw. US-PS 3912 752) bzw. lassen sich nach beschriebenen Verfahren leicht herstellen (sogenannte Williamson'sche Äthersynthese).

Angaben zur Darstellung der neuen Zwischenproduckte der Formel IV: Als Verdünnungsmittel kommen für die Umsetzung der Phenoxy-ketone der Formel (VIII) mit dem 3-Pyridinaldehyd der Formel (IX) vorzugsweise Wasser und inerte organische Lösungsmittel infrage. Hierzu gehören vorzugsweise Ether, wie Diethylether oder Tetrahydrofuran; Alkohole, wie Methanol oder Ethanol; sowie Dimethylsulfoxyd und Dimethylformamid. Vorzugsweise werden auch Mischungen von Wasser mit einem inerten organischen Lösungsmittel eingesetzt.

Als Basen kommen vorzugsweise infrage: tertiäre Amine, wie Triethylamin; Alkalicarbonate, wie Kaliumcarbonat, Alkalimetallhydroxide, wie Natriumhydroxid und Alkalimetallalkoholate, wie Natriummethylat.

Die Reaktionstemperaturen können in einem größeren Bereich variiert werden. Im allgemeinen arbeitet man zwischen -20 und 120°C, vorzugsweise zwischen 0 und 80°C.

Bei der Durchführung dieser Umsetzung setzt man auf 1 Mol Phenoxyketon der Formel (VIII) vorzugsweise 1 bis 2 Mol 3-Pyridinaldehyd ein. Die Isolierung der Verbindungen der Formel (IV) erfolgt in üblicher Weise (vergleiche auch die Herstellungsbeispiele).

Für die erfindungsgemäße Umsetzung gemäß Verfahren (a) kommen als Verdünnungsmittel vorzugsweise inerte organische Lösungsmittel infrage. Hierzu gehören vorzugsweise Ether, wie Diethylether; Alkohole, wie Methanol; Ketone, wie Aceton; aromatische Kohlenwasserstoffe, wie Benzol; sowie auch Dimethylsulfoxid und Dimethylformamid.

Die erfindungsgemäße Umsetzung gemäß Verfahren (a) wird in Gegenwart eines Säurebinders vorgenommen. Man kann alle üblicherweise verwendbaren anorganischen oder organischen Säurebinder zugeben, wie Alkalimetallcarbonate, beispielsweise Kaliumcarbonat oder Natriumcarbonat, Alkalimetallhydroxide, beispielsweise Natriumhydroxid, Alkalimetallalkoholate, beispielsweise Natriummethylat, oder wie niedere tertiäre Alkylamine, beispielsweise Triethylamin.

Die Reaktionstemperaturen können bei der Durchführung des erfindungsgemäßen Verfahrens (a) in einem größeren Bereich variiert werden. Im allgemeinen arbeitet man zwischen 0 und 140°C, vorzugsweise zwischen 50 und 100°C.

Bei der Durchführung des erfindungsgemäßen Verfahrens (a) setzt man auf 1 Mol der Verbindung der Formel (II) vorzugsweise 2 bis 4 Mol Phenol der Formel (III) ein. Die Isolierung der Verbindungen der Formel (I) erfolgt in üblicher Weise. Die Verbindungen der Formel (II) werden vorzugsweise in Form ihrer Hydrohalogenide eingesetzt.

Für die erfindungsgemäße Umsetzung gemäß Verfahren (b/α) kommen als Verdünnungsmittel polare organische Lösungsmittel infrage. Hierzu gehören vorzugsweise Alkohole, wie Methanol und Ethanol, sowie Nitrile, wie Acetonitril.

Die erfindungsgemäße Umsetzung gemäß Verfahren (b/α) wird in Gegenwart eines Katalysators vorgenommen. Vorzugsweise werden Edelmetall-, Edelmetalloxid- bzw. Edelmetallhydroxid-Katalysatoren oder sogenannte Raney-Katalysatoren verwendet, insbesondere Platin, Platinoxid und Nickel.

Die Reaktionstemperaturen können bei der Durchführung des Verfahrens (b/α) in einem größeren Bereich variiert werden. Im allgemeinen arbeitet man zwischen 0 und 50°C, vorzugsweise zwischen 20 und 40°C.

Die erfindungsgemäße Umsetzung gemäß Verfahren (b/α) kann bei Normaldruck, aber auch bei erhöhtem Druck, wie 1 bis 2 atü, durchgeführt werden.

Bei der Durchführung des erfindungsgemäßen Verfahrens (b/α) setzt man auf 1 Mol der Verbindung der Formel (IV) etwa 1 Mol Wasserstoff und 0,1 Mol Katalysator ein. Zur Isolierung der Verbindungen der Formel (I) wird vom Katalysator abfiltriert, vom Lösungsmittel im Vakuum befreit und die erhaltenen Produkte in üblicher Weise gereinigt.

Die Reaktionstemperaturen können bei der Durchführung des Verfahrens (b/ß) in einem größeren Bereich variiert werden. Im allgemeinen arbeitet man zwischen 50 und 150°C, vorzugsweise 80 und 120°C.

Bei der Durchführung des erfindungsgemäßen Verfahrens (b/ß) setzt man auf 1 Mol der Verbindung der Formel (IV) vorzugsweise 3 bis 4 Mol Metall ein. Die Isolierung der Verbindungen der Formel (I) erfolgt in üblicher Weise.

Die erfindungsgemäße Reduktion der Keto-Verbindungen der Formel I zu den entsprechenden sekundären Alkoholen erfolgt in üblicher Weise, wie z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Aethanol, Butanol, Isopropanol, und Aether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (I) etwa 1 Mol eines komplexed Hydrids, wie Natriumhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeiung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (I) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die tnstandene Aluminium-Verbindung mit verdünnter Schwefelsäure oder Natronlauge zersetzt. Die weitere Aufarbeitung erfolgt in üblicher Weise.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronsäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich von physiologisch verträglichen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung echter Mehltaupilze, wie zur Bekämpfung von Getreidemehltau, verwendet werden; außerdem auch gegen den Erreger des Apfelschorfs (Fusicladium dendriticum) sowie zur Bekämpfung der Pilze Pyricularia und Pellicularia.

Eine fungizide Wirkung zeigen außerdem auch die neuen Zwischenprodukte der Formel (IV).

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon. stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel A.

Sproßbehandlungs-Test / Getreidemehltau / protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykol-ether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.

Nach 6 Tagen Verweilzeit der Pflanzen bei einer Temperatur von 21 - 22°C und einer Luftfeuchtigkeit von 80 - 90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.

Bei diesem Test zeigen z.B. folgende Verbindungen eine sehr gute Wirkung, die derjenigen der aus dem Stand der Technik bekannten Verbindungen überlegen ist:
Verbindungen gemäß Herstellungsbeispielen: 4, 2, 5, 6, 7, 8 und 3.

Herstellungsbeispiele

Beispiel 1

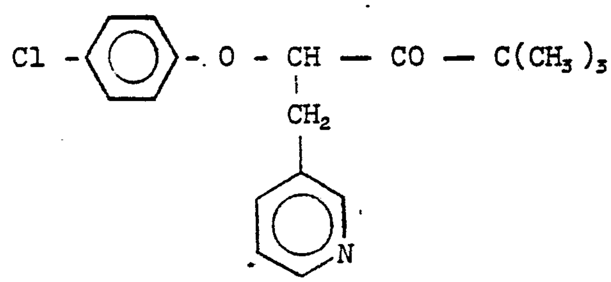

(Verfahren a)

38,6g (0,3 Mol) 4-Chlorphenol werden in 200 ml absolutem Methanol gelöst und mit 6,9g (0,3 Mol) Natrium versetzt. Nach vollständiger Auflösung des Natriums wird die Lösung 30 Minuten unter Rückfluß erhitzt, dann werden bei leichtem Sieden 35,1g (0,1 Mol) 2-Brom-4,4-dimethyl-1-pyridin-3-yl-pentan-3-on-hydrobromid auf einmal zugegeben. Die Reaktionsmischung wird über Nacht unter Rückfluß erhitzt und nach dem Abkühlen eingeengt. Der Rückstand wird in Ether und 2n Natronlauge aufgenommen, die Etherphase abgetrennt, zweimal mit 2n Natronlauge, einmal mit Wasser und einmal mit gesättigter Natriumchloridlösung gewaschen, danach über Natriumsulfat getrocknet und eingeengt. Nach Umkristallisation aus Cyclohexan erhält man 3,3g (11,5% der Theorie) 2-(4-Chlorphenoxy)-4,4-dimethyl-1-pyridin-3-yl-pentan-3-on vom Schmelzpunkt 76-78°C.

Herstellung der Vorprodukte

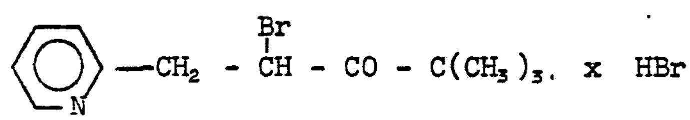

320,5g (1,676 Mol) 4,4-Dimethyl-1-pyridin-3-yl-pentan-3-on werden in 320 ml einer wässerigen 48%-igen Bromwasserstoffsäure

gelöst und bei 80 bis 95°C mit 267,8 g (85,3 ml; 1,65 Mol) Brom während 2 Stunden tropfenweise versetzt. Nach beendeter Zugabe wird die Lösung gekühlt und eingeengt. Der zurückbleibende Feststoff wird gut zerkleinert, mit ca. 1,5 l Ethanol verrührt, abgesaugt, mit Ethanol gewaschen und getrocknet. Man erhält 450g (78 % der Theorie) 2-Brom-4,4-dimethyl-1-pyridin-3-yl-pentan-3-on vom Schmelzpunkt 163-64°C.

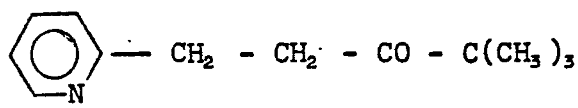

154,2g (0,814 Mol) 4,4-Dimethyl-1-pyridin-3-yl-1-penten-3-on werden in 750 ml Tetrahydrofuran gelöst und mit ca. 20g Raney-Nickel verrührt. Die Lösung wird filtriert, nochmals mit ca. 20g Raney-Nickel versetzt und bei gewöhnlichem Druck und Raumtemperatur hydriert, bis laut Dünnschichtchromatogramm kein Ausgangsprodukt mehr vorhanden ist. Die Lösung wird vom Raney-Nickel filtriert, eingeengt und der Rückstand destilliert. Man erhält 119,8g (78 % der Theorie) 4,4-Dimethyl-1-pyridin-3-yl-pentan-3-on vom Siedepunkt 123-130°C/2,3mm.

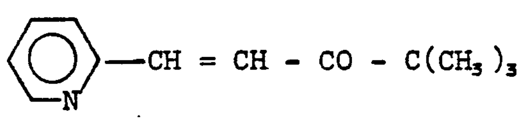

Zu einer Lösung von 35g (0,35 Mol) 3,3-Dimethyl-butan-2-on und 38g (0,355 Mol) 3-Pyridinaldehyd in 2000 ml Wasser werden 100 ml 10%-ige Natronlauge gegeben. und die Lösung über Nacht bei Raumtemperatur gerührt. Der kristallineNiederschlag wird abgesaugt, getrocknet und aus n-Hexan umkristallisiert. Man erhält 34,1g (51,5 % der Theorie) 4,4-Dimethyl-1-pyridin-3-yl-1-penten-3-on vom Schmelzpunkt 68-70°C.

(Verfahren b)

Zu 48g (0,152 Mol) 2-(4-Chlorphenoxy)-4,4-dimethyl-1-pyridin-3-yl-1-penten-3-on, gelöst in 170 ml Eisessig, werden 32g (0,49 Mol) Zinkstaub gegeben und die Mischung 40 Minuten auf einem Oelbad unter Rückfluß erhitzt. Die Lösung wird vom Zink abgesaugt, das Zink mit Eisessig nachgewaschen und die Essigsäurelösung eingeengt. Der Rückstand wird mit 1n Natronlauge alkalisch gestellt und dreimal mit Essigester extrahiert. Aus der Essigesterphase erhält man, nach Umkristallisation aus Cyclohexan, 29,9g (62 % der Theorie) 2-(4-Chlorphenoxy)-4,4-dimethyl-1-pyridin-3-yl-pentan-3-on vom Schmelzpunkt 77-79°C.

Herstellung des Ausgangsproduktes

Beispiel IV-1 (neues Zwischenprodukt der allgemeinen Formel IV):

(IV-1) 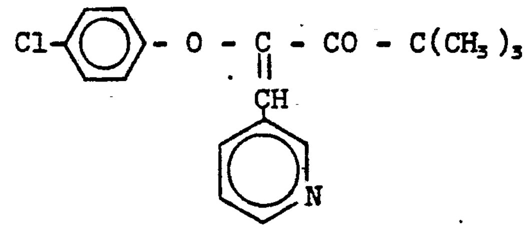

Zu einer gerührten Lösung von 22,6g (0,1 Mol) 1-(4-Chlorphenoxy)-3,3-dimethyl-butan-2-on und 21,4g (0,2 Mol) 3-Pyridinaldehyd in 500 ml Methanol werden 100 ml Wasser gegeben, gefolgt von 28 ml 10%-iger wässriger Natronlauge. Die Lösung wird 4 Tage bei Raumtemperatur nachgerührt, das Methanol im Vakuum entfernt und der wässrige Rückstand in Ether aufgenommen. Die wässrige Phase wird abgetrennt, die organische Phase dreimal mit 1n-Salzsäure extrahiert, die Salzsäureextrakte mit festem Natriumhydrogencarbonat neutralisiert und dreimal mit Essigester extrahiert. Aus der Essigesterphase erhält man 22,8 g (72 % der Theorie) 2-(4-Chlorphenoxy)-4,4-dimethyl-1-pyridin-3-yl-1-penten-3-on vom Schmelzpunkt 74-76°C.

Beispiel 2

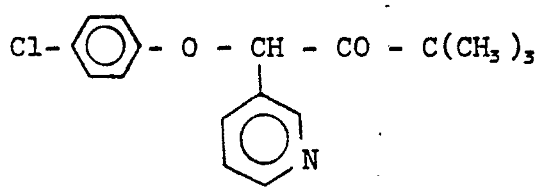

(Verfahren a)

Eine Lösung von 38,6g (0,3 Mol) 4-Chlorphenol wird in 200 ml absolutem Methanol mit 6,9g (0,3 Mol) Natriummetall versetzt. Nach vollständiger Auflösung des Natriums wird die Lösung unter Rückfluß erhitzt und mit 33,7g (0,1 Mol) 1-Brom-3,3-dimethyl-1-pyridin-3-yl-butan-2-on-hydrobromid versetzt. Die Reaktionsmischung wird über Nacht unter Rückfluß erhitzt. Man läßt abkühlen und engt im Vakuum ein. Der Rückstand wird in Diethylether aufgenommen, zweimal mit 2n-Natronlauge, einmal mit Wasser und einmal mit gesättigter Natriumchloridlösung gewaschen, über Natriumsulfat getrocknet und eingeengt. Der Rückstand wird aus n-Hexan umkristallisiert. Man erhält 18,8g (62 % der Theorie) 1-(4-Chlorphenoxy)-3,3-dimethyl-1-pyridin-3-yl-butan-2-on vom Schmelzpunkt 50°C.

Beispiel 3

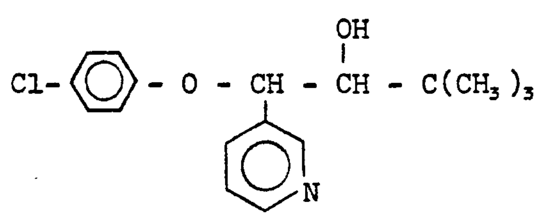

(Reduktion)

Eine Lösung von 15,2g (0,05 Mol) 1-(4-Chlorphenoxy)-3,3-dimethyl-1-pyridin-3-yl-butan-2-on (Beispiel 2) in 100ml absolutem Ethanol wird mit 1,9g (0,05 Mol) Natriumborhydrid versetzt. Die Reaktionsmischung wird über Nacht

gerührt, danach mit Wasser und etwas verdünnter Salzsäure versetzt. Man engt durch Abdestillieren des Ethanols im Vakuum ein. Der wässrige Rückstand wird mit einer gesättigten Natriumhydrogencarbonat-Lösung alkalisch gestellt und dreimal mit Essigester extrahiert. Die vereinigten Essigesterphasen werden über Natriumsulfat getrocknet und eingeengt. Nach Umkristallisation des Rückstandes aus Cyclohexan erhält man 7,2g (47 % der Theorie) 1-(4-Chlorphenoxy)-3,3-dimethyl-1-pyridin-3-yl-butan-2-ol vom Schmelzpunkt 116-117°C.

In analoger Weise werden die folgenden Verbindungen der allgemeinen Formel

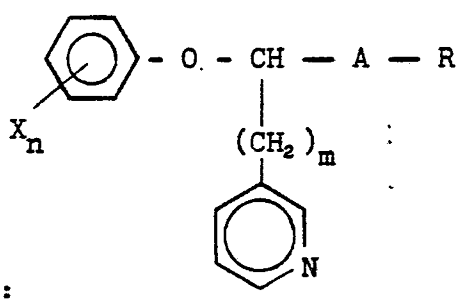

(I)

erhalten:

| Bsp. Nr. | $X_n$ | A | R | m | Schmelzpunkt (°C) |
|---|---|---|---|---|---|
| 4 | 2,4-$Cl_2$ | CO | $C(CH_3)_3$ | 0 | 74 |
| 5 | 2-$CH_3$ | CO | $C(CH_3)_3$ | 0 | 212 (x1/2NDS) |
| 6 | 2-$CH_3$,4-Cl | CO | $C(CH_3)_3$ | 0 | 226-27(x1/2NDS) |
| 7 | 2-Cl | CO | $C(CH_3)_3$ | 0 | 230-31(x1/2NDS) |
| 8 | 4-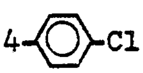-Cl | CO | $C(CH_3)_3$ | 0 | 131 |

| Bsp. Nr. | $X_n$ | A | R | m | Schmelzpunkt (°C) |
|---|---|---|---|---|---|
| 9 | 2,4-$Cl_2$ | CO | 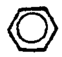 | 0 | 151-53 |
| 10 | 2-$CH_3$,4-Cl | CO | 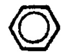 | 0 | 137-38 |
| 11 | 3,4-$Cl_2$ | CO | $C(CH_3)_3$ | 1 | 87-88 |
| 12 | 2-$CH_3$ | CO | $C(CH_3)_3$ | 1 | 198-200(x1/2NDS) |
| 13 | 2,4-$Cl_2$ | CO | $C(CH_3)_3$ | 1 | 212-14(x1/2NDS) |
| 14 | 4-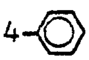-Cl | CO | $C(CH_3)_3$ | 1 | 93-94 |
| 15 | 2-Cl | CO | $C(CH_3)_3$ | 1 | 217(x1/2NDS) |
| 16 | - | CO | $C(CH_3)_3$ | 1 | 52-54 |
| 17 | 4-$CH_3$ | CO | $C(CH_3)_3$ | 1 | 54-56 |
| 18 | 2-$CH_3$,4-Cl | CO | $C(CH_3)_3$ | 1 | 199-201(x1/2NDS) |
| 19 | 4-O-$CH_2$-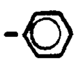 | CO | $C(CH_3)_3$ | 1 | 113-14 |

NDS = 1,5-Naphthalindisulfonsäure

Entsprechend Beispiel IV-1 werden die neuen Zwischenprodukte der allgemeinen Formel

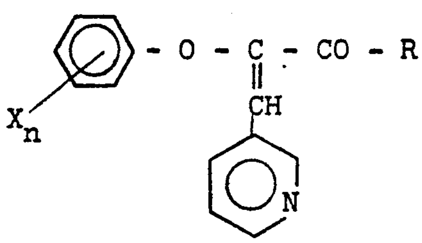

(IV)

erhalten:

| Bsp.Nr. | $X_n$ | R | Schmelzpunkt (°C) |
|---|---|---|---|
| IV-2 | 2-$CH_3$ | $C(CH_3)_3$ | 91-93 |
| IV-3 | 2,4-$Cl_2$ | $C(CH_3)_3$ | 253-55(x1/2NDS) |

| Bsp.-Nr. | $X_n$ | R | Schmelzpunkt (°C) |
|---|---|---|---|
| IV-4 | 3,4-$Cl_2$ | $C(CH_3)_3$ | 85-86 |
| IV-5 | 4-F | $C(CH_3)_3$ | 71-73 |
| IV-6 | 4-$OCH_3$ | $C(CH_3)_3$ | 87-88 |
| IV-7 | 2-Cl | $C(CH_3)_3$ | 71-73 |
| IV-8 | 2-$CH_3$,4-Cl | $C(CH_3)_3$ | 249-51(x1/2NDS) |
| IV-9 | - | $C(CH_3)_3$ | 46-48 |
| IV-10 | 2,4-$Cl_2$ | 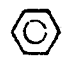 | 117-18 |
| IV-11 | 4-F | 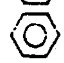 | 98-100 |
| IV-12 | 2-$CH_3$,4-Cl | 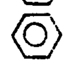 | 118-20 |
| IV-13 | - | 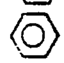 | 112-14 |
| IV-14 | 4-$CH_3$ | 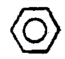 | 80-81,5 |
| IV-15 | 4-$OCH_3$ | 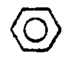 | 96,5-98 |
| IV-16 | 2-$CH_3$ | 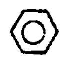 | 132-33 |
| IV-17 | 2-$C_2H_5$ | 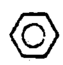 | 65-66 |
| IV-18 | 4-0-$CH_2$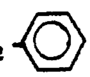 | $C(CH_3)_3$ | 172-75(x1/2NDS) |

NDS = 1,5-Naphthalindisulfonsäure

Patentansprüche:

1) 3-Substituierte Pyridin-Derivate der allgemeinen Formel

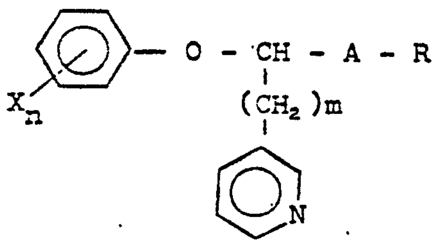

(I)

in welcher

A für die -CO- oder die -CH(OH)-Gruppe steht,

R für Alkyl oder gegebenenfalls substituiertes Phenyl steht,

X für Halogen, Alkyl, Cycloalkyl, Alkoxy, Alkylthio, Halogenalkyl, gegebenenfalls substituiertes Phenyl, gegebenenfalls substituiertes Phenoxy, gegebenenfalls substituiertes Phenylalkyl oder gegebenenfalls substituiertes Phenylalkoxy steht,

n für ganze Zahlen von 0 bis 3 steht und

m für 0 oder 1 steht,

sowie deren physiologisch verträgliche Säureadditions-Salze und Metallsalz-Komplexe.

2) Verfahren zur Herstellung von 3-substituierten Pyridin-Derivaten der Formel I, dadurch gekennzeichnet, daß man

a) Halogen-pyridin-ketone der Formel

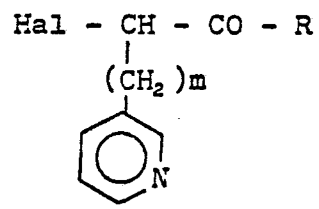

(II)

in welcher

R und m die oben angegebene Bedeutung haben und

Hal für Halogen, insbesondere Chlor oder Brom steht,

mit Phenolen der Formel

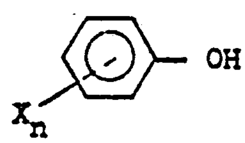

(III)

in welcher

X und n die oben angegebene Bedeutung haben,

in Gegenwart eines Verdünnungsmittels und in Gegenwart eines Säurebindemittels umsetzt, oder

b) α, ß-ungesättigte Ketone der Formel

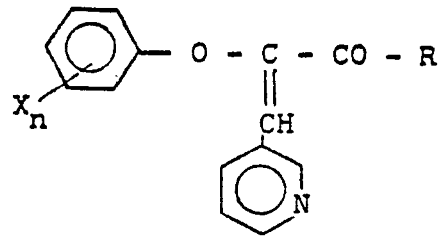

(IV)

in welcher

R,X und n die oben angegebene Bedeutung haben,

in an sich bekannter Art und Weise

α) mit Wasserstoff in Gegenwart eines Katalysators und gegebenenfalls in Gegenwart eines polaren Verdünnungsmittels oder

ß) mit einem Metall/Säure-Gemisch, wie insbesondere Zink/Eisessig umsetzt und

gegebenenfalls die nach den Verfahrensvarianten (a) und (b) erhaltenen Keto-Derivate der Formel I nach bekannten Methoden in üblicher Weise zu den sekundären Alkoholen der Formel I reduziert,
und gegebenenfalls anschließend eine Säure oder ein Metallsalz addiert.

3) Fungizide Mittel, gekennzeichnet durch einen Gehalt an mindestens einem 3-substituierten Pyridin-Derivat der Formel I.

4) Verfahren zur Bekämpfung von Pilzen, dadurch gekennzeichnet, daß man 3-substituierte Pyridin-Derivate der Formel I auf Pilze oder ihren Lebensraum einwirken läßt.

5) Verwendung von 3-substituierten Pyridin-Derivaten der Formel I zur Bekämpfung von Pilzen.

6) Verfahren zur Herstellung von fungiziden Mitteln, dadurch gekennzeichnet, daß man 3-substituierte Pyridin-Derivate der Formel I mit Streckmitteln und/oder oberflächenaktiven Mitteln vermischt.

7) α,β-ungesättigte Ketone der allgemeinen Formel

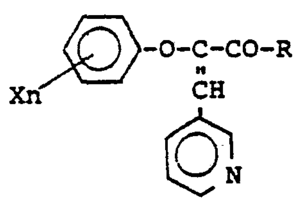

(IV)

in welcher
R, X und n die oben angegebene Bedeutung besitzen.

Europäisches Patentamt

# EUROPÄISCHER RECHERCHENBERICHT

Nummer der Anmeldung
EP 80 10 1041

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P,A | DE - A - 2 742 175 (BAYER A.G.)<br>* Ansprüche *<br>-- | |
| E | GB - A - 2 015 524 (IMPERIAL CHEMICAL INDUSTRIES LTD.)<br>* Ansprüche *<br>---- | 1,3 |

KLASSIFIKATION DER ANMELDUNG (Int.Cl. 3)

C 07 D 213/30
213/50
A 01 N 43/40

RECHERCHIERTE SACHGEBIETE (Int. Cl. 3)

C 07 D 213/30
213/50

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 09-06-1980 | VAN BIJLEN |